# EUROPEAN PATENT SPECIFICATION

(11) **EP 1 776 157 B1**
(45) Date of publication and mention of the grant of the patent: **29.06.2011**
(21) Application number: 05788946.1
(22) Date of filing: 12.07.2005
(51) Int. Cl.: A61M 37/00

(54) **PACKAGING AND METHOD FOR SOLID DOSE ADMINISTRATION OF AN ELECTRONIC IDENTIFICATION CHIP AND MEDICAMENTS**
VERPACKUNG UND VORRICHTUNG ZUR ABGABE VON FESTEN DOSIERUNGEN EINES ELEKTRONISCHEN IDENTIFIKATIONS-CHIPS UND MEDIKAMENTEN
EMBALLAGE ET MÉTHODE D'AMINISTRATION DE DOSE SOLIDE D'UNE PUCE D'IDENTIFICATION ÉLECTRONIQUE ET DE MÉDICAMENTS

(30) Priority: 12.07.2004 US 889558
(43) Date of publication of application: 25.04.2007
(73) Proprietor: Solidtech Animal Health, Inc., New Castle, OK 73065 (US)
(72) Inventor: HANSEN, Richard, D., New Castle, OK 73065 (US); ANDERSON, Mark,L., New Castle, OK 73065 (US)
(74) Representative: Tomkinson, Alexandra
(86) International application number: PCT/US2005/024736
(87) International publication number: WO 2006/017308

(56) References cited:
- EP-A- 0 440 998
- US-A- 4 004 565
- US-A- 4 077 406
- US-B1- 6 530 896

## Description

### FIELD OF THE INVENTION

This invention relates to inoculation of living animals, usually companion animals, livestock or wildlife, with an electronic identification chip and a biologically active solid dose pellet. They may be administered subcutaneously as implants, or ballistically as a projectile.

### BACKGROUND OF THE INVENTION

Pellet implant systems which administer hormonal medicaments subcutaneously are known. Typically, they use a dosing gun to administer a series of pharmaceutical pellet implants, usually to the ear of an animal to provide sustained release of medicament. A dosing gun or implanter usually incorporates pellet magazines containing multiple doses of pharmaceutical pellets which are inserted subcutaneously into the ear tissue with an associated injection needle. The magazine channels are loaded with a series of identical pharmaceutical pellets for administration at the same injection site.

Implant technology, that is to say, procedures involving subcutaneous implants of pharmaceuticals are now well accepted and widespread in the areas of animal health and production enhancement. Growth stimulants are commonly used to enhance the body weight of animals which are raised for slaughtering, such as cattle, swine, sheep, turkeys, chickens and the like. A medicament in the pellet is normally formulated for continuous sustained absorption of the active ingredients over an extended period of time.

Solid dose ballistic projectiles shaped for penetrating the epidermal layer of living animal tissue are also known. These are typically fired remotely, using an airgun. They lodge totally within the tissues of the animal for later release of biologically-active medicaments into the animal tissue. They have an advantage over typical hand-held implant apparatus using a needle in that the inoculated animal need not be "captured".

This invention may be used with either type of solid dose administration, but has been found particularly useful with hand-held implant administration, more particularly where combined dosing of a plurality of biologically active materials is achieved with a single needle-inserted, subcutaneous implantation. For details of such, implant systems, see my previous patent, U.S. Patent 5,665,363, issued September 9, 1997, and as well, other prior patents on pellet implant systems, such as U.S. Patent 5,874,098, issued February 23, 1999 and U. S. Patent No. 6,290,980 issued September 18, 2001. Disclosures of each of these are incorporated herein by reference.

Implant systems are also known for use in animal identification. That is to say passive electronic devices that emit a radio frequency when activated by a scanner are becoming increasingly popular, particularly for companion pet identification. These electronic identification chips can be as small as a rice-sized chip, and operate at frequencies of between 100 kHz to 400 kHz, particularly preferably is 125 kHz and 134.2 kHz. Such devices are commercially available from U.S. Companies AVID, DESTRON Technologies, and the Swiss-based company DATAMARS. Since they are commercially available and in some instances patented, they do not need to be described herein in detail. For examples of construction and operation of electronic identification chips that may be implanted in companion animals, see Beigel - U.S. Patents 4,333,072 issued June 1, 1982; Beigel - U.S. Patent 5,214,409 issued May 25, 1993; Beigel et al. - U.S. Patent 5,235,326 and the patent of Harmon et al. - U.S. Patent 5,772,671, the disclosures of which are incorporated by reference. While the greatest popularity of these electronic identification chips has in the past been with companion animals, their use in animal monitoring can also include domesticated livestock, poultry and wild life.

While others have in the past recognized the advantages of biologically-active medicinal implants (see patent of co-inventor Hansen, 6,290,980 and of co-inventor Anderson 5,772,671) and separately recognized use of electronic identification chip implants (see e.g. 5,235,326) for reasons unknown to the present inventors, no-one has thought of combining both technologies. Doing so allows single dosing in an effort to not only monitor the animal, but monitor the precise medicaments the animal gets, but to also allow packaging all in the same package while dosing both simultaneously resulting in less labor and less stresses to the animal. It is one of the primary objects of this invention to achieve these advantages in a single system which doses both an implant identification chip and a medicament system, at once.

US4004565 discloses a dosing pellet magazine comprising a plurality of see through pellet dosing columns. Each of the columns is loaded with a pellet being coded to represent a biologically active medicament.

US6530896 (closest prior art) discloses an implant cartridge for insertion of time release drug implants or micro electronic chip implants. Particularly, when animals are multiply dosed, it is necessary for the medicament administrator to keep track of medicaments, and of which animals have in fact been dosed. This can be particularly bothersome when multiple dosing of medicament occurs with a single subcutaneous implant, or shot, in the case of a ballistic implant. This invention has as its objective a new method, packaging and system for keeping track of animals and systematically administering a plurality of medicaments to animals along with an identification chip so that the operator, or medicament administrator, can be confident that all desired medicaments have in fact been administered and so the animals location can be tracked.

### SUMMARY OF THE INVENTION

According to the present invention there is provided a dosing pellet magazine for use in inoculating an animal, comprising; a plurality of connected pellet dosing columns, each of said columns being loaded with an electronic identification microchip and at least one biologically active dosing pellet, with said pellet being coded to represent a particular biologically active medicament, characterised in that the microchip has a convex end that is complementary to a concave surface of the at least one biologically active dosing pellet provided next to and behind the microchip in the column.

A method and packaging for both implanting medicaments and an identifying chip for vaccinating or medicating an animal by implanting a solid dose medicament in an animal. The solid dose medicament, or plurality of such medicaments, are each color-coded or otherwise coded by surface identification to identify a particular active ingredient or ingredients, then packaged in a pellet magazine, syringe body or affixed needle through which the doses may or may not be visible, and thereafter the pellets are implanted, usually subcutaneously into the base of the ear of in the neck or flank of an animal. The same system can be used for colored implants administered ballistically. The animal can thereafter be monitored because of the identifying microchip.

### BRIEF DESCRIPTION OF THE DRAWINGS

Figure 1 is a front view of a bovine head, showing the ears.
Figure 2 is a back view of a bovine head for illustrative purposes, showing the subcutaneous biological ear implant site.
Figure 3 is a side view of a solid dosing implanter.
Figure 4 shows a typically pellet magazine in perspective.
Figure 5 shows a pellet magazine which is transparent, showing how the microchip (M) and pellets are arranged in a prearranged multiple dosing order.
Figure 6 shows how the prearranged dosing order for the pellets can be color-coded or otherwise coded.
Figure 7 shows an optional device for implanting.

### DESCRIPTION OF THE PREFERRED EMBODIMENT

While the description here is given as a preferred embodiment of the invention, it is to be understood that the invention is not limited to the preferred embodiment. Rather, the invention is limited only by the defining limits of the claims, as opposed to any statements in the specification relating to the preferred embodiment.

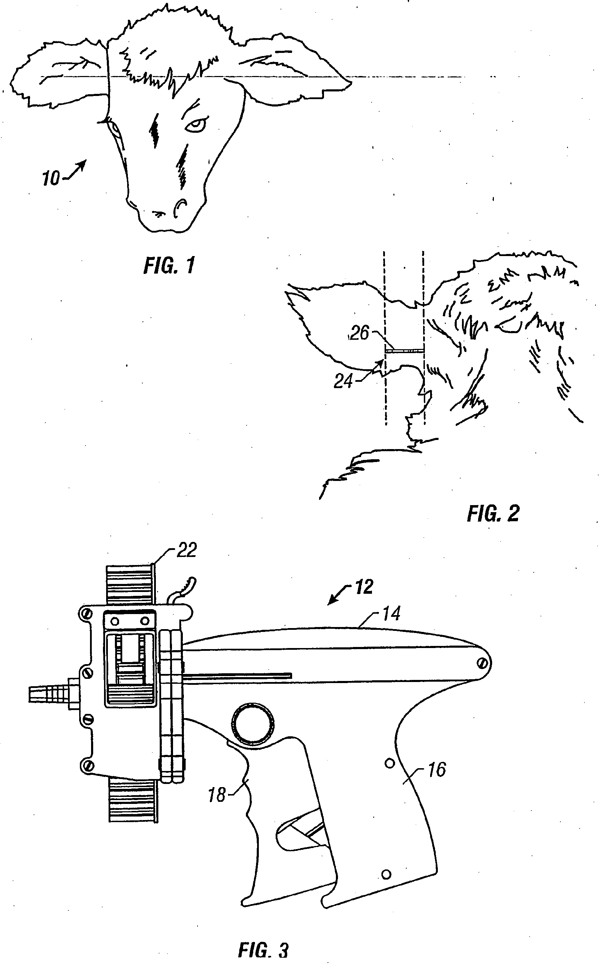

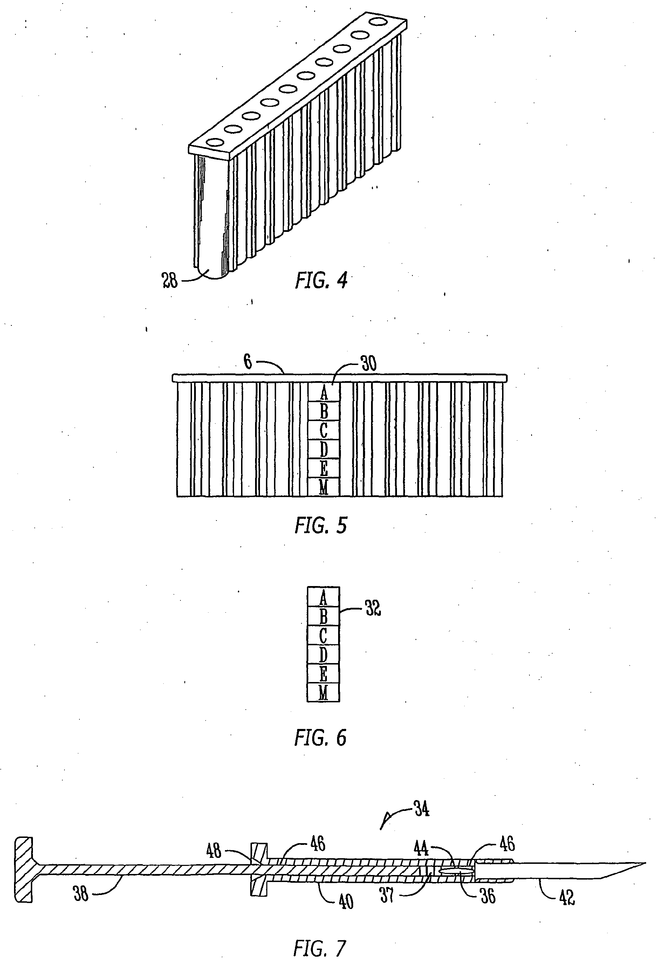

A calf 10 is indicated for illustrative purposes of showing the working of the implant system. A suitable solid dosing implanter 12 of known construction can be used. Such devices generally include a housing 14 with a pistol grip 16, a trigger 18, and a subcutaneous implant needle. The implant magazine 22 is inserted into the implanter apparatus 12, and moves downwardly therethrough as trigger 18 is pulled, unloading its identification chip (m) and biological or pharmaceutical dose through a needle as trigger 18 is pulled. This action forces the multiple doses through the bore of the needle (not depicted) attached to the implant apparatus 12, and into a subcutaneous puncture, particularly in the base of the ear 24 as illustrated at 26, Figure 2, or in the neck. For details of such a method, see my earlier patent 5,665,363. For dogs they most likely will be injected subcutaneously on the scruff of the neck using a syringe type implanter (Figure 7).

Dose magazine 22 is illustrated in perspective in Figure 4 and in Figure 3 as it would enter from the top and exit below the implanter apparatus 12. It can be seen that pellet magazine is made of an inert, see-through polymeric plastic material. The magazine 22 used with implanter 12 typically contains multiple, parallel-aligned, dosing columns or chambers 28. As illustrated, the chambers are generally of such a construction that each has a hollow internal core. The chambers 28 are in side-by-side parallel relation, as illustrated.

Each chamber 28 is loaded with an identification chip and a plurality of discrete dosing pellets in columnar relationship, as illustrated in Figure 5 at 30. Individual pellets in similar stacked relationship are shown in Figure 6. The individual pellets 32 are composed of an identified biologically active ingredient or multiple ingredients in conjunction with one or more excipients formed as part of a polymeric base release system such as described in my earlier U.S. Patent 5,665,363.

Figure 7 shows implanter 34 with an implant pellet 36, which could be either an electronic chip or the medicament, and push rod 38 installed in body 40 having a needle 42. Three friction ribs 44 located proximally of needle 42 on the interior wall 46 hold implant chip 36 and pellets 37 in place. Implant pellet 36 and medicament pellets are inserted into opening 48 and pushed along the interior wall 46 until it engages ribs 44. For further details see U.S. Patent 5,772,671 which is incorporated herein by reference. Multiple medicament pellets may be used within the syringe body.

Each of the solid dose pellets 32 include a wide range of biologically active medicaments. Indeed, the particular medicament used for pellet 32 is not a limitation on the invention. For example, the medicaments may be hormones, minerals, vitamins, antibiotics, antigens, antibodies, tranquilizers, dewormers, etc. The pellets are loaded behind the electronic identification chip which is first in the sequence.

Preferably, the biologically active pellet comprises about 2% to 70% by weight of a medicament. More preferably, the biologically active pellet comprises about 3% to 50% by weight of the medicament, most preferably about 4% to 20% by weight.

Typically, the dried powder mixture is blended with a lubricant and pelletized into final form. Lubricants facilitate the release of the pellets from the pelleting dies.

A list of lubricants that can be used in the practice of the invention includes, but should not be limited to, magnesium stearate, calcium stearate, sodium stearyl fumarate, stearic acid, sodium lauryl sulfonate, polyoxyethylene (carbowaxes), polyethylene glycols, glycerol behenate, hydrogenated vegetable oils and mixtures thereof. Preferably, the lubricant contains calcium stearate.

Generally, the biologically active pellet comprises an effective pellet-forming amount of a lubricant. Preferably, the biologically active pellet comprises about 0.2% to 5% by weight of a lubricant. More preferably, the biologically active pellet comprises about 0.5% to 3.5% by weight of a lubricant, most preferably about 1.0% to 2% by weight.

**TABLE 1**

| | % By Weight of the Biologically Active Pellet | | |
|---|---|---|---|
| | Working Range | Preferred Range | Most Preferred Range |
| Biologically Active Material | 2%-70% | 3%-50% | 4%-20% |
| Excipients | balance | balance | balance |
| Lubricant | 0.2%-5% | 0.5%-3.5% | 1%-2% |

Optionally, the biologically active pellet can comprise additional excipients. These additional excipients can be added to the biologically active pellet to provide increased strength, to control dissolution rates, to improve powder handling, e.g., flow and the like, or to improve the efficacy of the product. A list of additional excipients that can be used as the biologically active pellet of the invention includes, but should not be limited to: precipitated or fumed silicas, sodium starch glycolates, calcium phosphate, calcium carbonate, dextrins, polyvinyl pyrrolidone, hydroxypropylcellulose, hydroxypropylmethylcellulose, polylactic acid, polyglycolic acid, magnesium aluminum silicates, microcrystalline cellulose, sodium carboxymethylcellulose and mixtures thereof. Preferably, these additional excipients are less than about 50% by weight of the biologically active pellet. More preferably, these additional excipients constitute less than about 40% by weight of the biologically active pellet, most preferably 25% by weight.

Pellets may be generally prepared as follows:
A liquid suspension containing, for example, bacterial cells and associated products adsorbed on aluminum hydroxide gel is mixed with sufficient mannitol to yield a final weight of 15 milligrams per dose of product. The suspension is dispensed into containers, frozen, and the water removed under vacuum. After freeze drying is complete, the dried powder is harvested. The powder is processed to reduce the particle size to less than 0.1 millimeter, and sufficient calcium stearate (approximately 1% by weight) is added for lubrication.

The powder blend is then tabletted on a conventional tableting machine to produce uniform pellets. A typical formulation for the above pellets would be:

| | |
|---|---|
| Freeze-dried powder | 15 |
| Calcium stearate | 2 |
| Precipitated silica | 0.5 |
| Filler, | balance |

| | |
|---|---|
| (Powder has 10 parts bacterial antigen and 5 parts mannitol) | |

Pellets produced as above may, if desired, be converted to delayed release pellets by coating with materials that will delay the escape of the medicament to the body. Materials or incorporating materials that are useful for this are compounds that will slowly degrade or dissolve in the body fluids. Examples of materials suitable for use are hydrolytically unstable polymers such as polylactic acid, polymers such as ethylvinyl acetate that are slow to dissolve in body fluids, or waxy solids, such as cholesterol, that have a limited solubility in aqueous fluids. These materials can be applied to the tablet as coatings and will act to delay the release of the active ingredient from the pellet.

There are a number of coating techniques available for adding the delayed release coatings to the pellets. Rotating drum coaters or fluidized bed coating processes can be used. Any process that can apply a uniform coating in a controlled manner can be used. The thickness of the coating and water solubility will determine the delay before the product is released.

The biologically active pellets of the invention can be formed into any possible shape that the pelletizing machine is capable of making. Preferably, the shape and size of the biologically active pellet is one suitable for implanting into the animal. More preferably, the shape of the biologically active pellet is such that it can be used in conjunction with an implant gun. Most preferably, the shape and size of the pellet is adapted for implanting the biologically active pellet subcutaneously into an ear or neck of the animal.

In general, the size of the biologically active pellet depends on the dose to be administered to the animal and compatibility with the implant gun and needle used. The electronic identification microchip is available in varying sizes. Some of those available are as small as a rice-sized chip. However, it is preferred that the chip and the biologically-active medicament pellet be of the same relative dimensions. Since it is easier to manage sizing for the medicament than electronic identification chip, the medicament is generally made in a size and volume relationship such that it will be of the same or similar size as the purchased electronic identification chip. This is for ease of packaging in a distribution magazine, for example of the type shown in Figure 5, or in the syringe barrel for an administration syringe of the type shown in Figure 7. The biological pellet next to the electronic microchip may be made with a concave surface to complement the convex end of the microchip in order to help prevent crushing the medicament pellets during delivery.

The biologically active pellets of the invention can be implanted into any animal which is capable of responding to the biologically active material. Generally, these animals include, but should not be limited to, cattle, hogs, horses, cats, dogs, sheep, goats, for example. In a preferred embodiment of the invention, the animal is domestic cattle.

The biologically active pellet can be subcutaneously implanted into any area of the animal which allows the biologically active pellet to come into contact with tissue fluids. Preferably, the biologically active pellet is implanted into an area of the animal which minimizes or eliminates lasting damage to edible tissue of the animal. More preferably, the biologically active pellet is implanted into an ear, the neck, the tail-head or flank areas of the animal, thereby reducing potential damage to edible tissue. Most preferably, the biologically active pellet is implanted into the ear of cattle, thereby eliminating damage to edible tissue.

In an earlier patent, co-inventor Hansen, found that implanting the biologically active pellet into an ear of the animal does not result in an undesirable "drooped ear", or "down ear" in the animal. As with any product administered through the skin, sanitary methods must be followed to reduce the likelihood of injection site infections/abscesses which may result in undesirable local tissue reactions.

The plurality of medicaments, if used, may be arranged in any predetermined order. One may arbitrarily select colors to represent each medicament pellet in the stack. For example, one vaccine could be represented by yellow, a second vaccine by blue, a third vaccine by white, a bacterin by green, and a second bacterin by red. These would be stacked in the order shown in Figure 6 so that looking through the columns 30 in Figure 5 one would see in this order from top to bottom yellow, blue, white, green, red. In all customer literature accompanying the dosing pellet magazine 28, the same color scheme would be consistently used throughout the description which accompanies the medicaments.

While the description herein has been given particularly with hand-held implant dosing, ballistic implants can be used of the type disclosed in my "MEDICAMENT DOSING BALLISTIC IMPLANT OF IMPROVED ACCURACY", Patent No. 6,375,971. If a similar color scheme is used with various medicaments, the implant casing itself could incorporate the color scheme as opposed to the payload. In like manner, the packaging could use consistent representations with regard to color being coordinated with specific medicaments. Such a ballistic implant would usually only contain a single medicament. Thus, for example, if an implant casing were yellow, one would know it contained a certain vaccine; if it were blue, one would know it contained another vaccine; red a certain bacterin; and so forth.

Use of the colorants is nonlimiting, but generally they would include FD&C colorants known to be non-harmful to the animals to be vaccinated, substantially inert to the administered biologically active material, and leave no persistent tissue discoloration.

The following were tested and found "non-viricidal" to the principal cattle modified live viruses (MLV) one might include in cattle vaccines. These include: Infectious Bovine Rhinotracheitis (IBR) Virus, Bovine Virus Diarrhea (BVD) Virus, Parainfluenza 3 (PI-3) Virus and Bovine Respiratory Syncytial Virus (BRSV): FD&C Red #40 aluminum lake pigment, FD&C Blue #1 aluminum lake pigment, FD&C Yellow #5 aluminum lake pigment arid the green combination of FD&C Blue #1 and FD&C Yellow #5 aluminum lake pigments.

These pigments in pellets were also found to leave no visible residues in the subcutaneous tissue of rabbits, cattle and swine. These pigments were accepted as satisfactory by the USDA-CVB (Center for Veterinary Biologics) and the FSIS (Food Safety and Inspection Service) for use in domestic cattle and swine."

It is likely the above actually tested list could be expanded to include other 21 CFR listed FD&C and D&C aluminum lake pigments, as well as other colorants (e.g., iron oxides, phenol red). Those next likely candidates include: FD&C Yellow #6 aluminum lake pigment, FD&C Red #40Y, FD&C Blue #2.

Color candidates may also be used in combination to create other colors (e.g., green).

The list of medicaments includes only veterinary products approved to be administered by the parenteral route. Generally, the products could include antigens related to: Vaccines (Live or Killed Virus), Bacterins and Bacterial Extracts (Killed organisms), Bacterin-Toxoids (Killed organisms + modified toxins), Toxoids (Modified toxins devoid of killed bacterial cells), and antibodies related to: Antisera (Serum containing antibodies against an infectious organism). Pharmaceuticals could also be used.

In practice, products could be prepared for all domestic and wild species. Specifically, products could include antigens or antibodies related to the following for cattle: Infectious Bovine Rhinotracheitis (IBR) virus, Bovine Virus Diarrhea (BVD) virus, Parainfluenza 3 (PI3) virus, Bovine Respiratory Syncytial Virus (BRSV), *Haemophilus, Mannheimia, Pasteurella, Leptospira, Clostridium, Campylobacter, Corynebacterium, Escherichia, Moraxella, Salmonella, Actinomyces, Anaplasmosis,* Anthrax, *Brucella,* Coronavirus, Rotavirus, *Staphylococcus, Trichomonas, Fusobacterium.*

For dogs, the following could be used: Distemper, Measles, Adenovirus, Parainfluenza, *Leptospira, Bordetella,* Parvovirus, Coronavirus, *Borrelia,* Rabies, *Salmonella.*

For horses, the following could be used: Anthrax, *Escherichia, Ehrlichia,* Encephalomyelitis, Influenza, Rabies, Rhinopneumonitis, *Salmonella,* Tetanus, Viral Arteritis, West Nile Virus.

For cats, the following could be used: Rhinotracheitis, Calicivirus, Panleukopenia, Leukemia, Rabies, Infectious Peritonitis, *Microsporum.*

For sheep, the following could be used: *Clostridium, Corynebacterium, Escherichia, Pasteurella, Salmonella,* Anthrax, *Bacteroides,* Bluetongue, *Campylobacter, Chlamydia,* Ecthyma, Epididymitis, Rabies, Tetanus.

For swine, the following could be used: Anthrax, *Bordetella, Clostridium, Corynebacterium, Escherichia,* Encephalomyocarditis, Erysipelas, *Actinobacillus, Haemophilus, Leptospira, Mycoplasma, Pasteurella,* PRRS, Influenza, Parvovirus, Pseudorabies, Rotavirus, Tetanus, *Salmonella, Streptococcus, Servulina,* Transmissible Gastroenteritis.

For avian, the following could be used: Bronchitis, Bursal Disease, *Mycoplasma,* Newcastle Disease, *Pasteurella,* Reovirus, *Bordetella,* Coccidiosis, *Escherichia,* Encephalomyelitis, *Erysipelas,* Fowl Pox, *Haemophilus,* Adenovirus, Herpesvirus, Influenza, Laryngotracheitis, Pacheco Disease, Paramyxovirus, *Salmonella.*

In addition, as earlier explained, the pellets, besides including the active which may be an antigen as above-described, and the colorant, they may contain other materials known in the tableting art. They may include fillers (inert), and fillers which are functional. Inert fillers include the following: Lactose, Mannitol, Dextrate, Dextrose, Fructose, Sucrose, Galactose, Maltose, Sorbitol, Dextran, Dextrin, Calcium carbonate, Calcium sulfate, Dicalcium phosphate. Functional fillers include the following: Alginic acid, Various cellulosics (Hydroxypropyl cellulose (HPC), Hydroxypropyl methylcellulose (HPMC), Oxidized cellulose (OC), Microcrystalline cellulose (MCC), Ethyl cellulose (EC), Hydroxyethyl cellulose (HEC), Methyl cellulose (MC), Carboxymethyl cellulose (CMC), Cellulose acetate (CA), Cellulose acetate butyrate (CAB), Cellulose acetate propionate (CAP), Cellulose sodium phosphate (CSP), Cellulose triacetate (CTA), Cellulose acetate phthalate

(C-A-P), Hydroxypropyl methylcellulose phthalate (HPMCP), Cellulose acetate trimellitate (C-A-T), Hydroxypropyl methylcellulose acetate succinate (HPMCAS), Sodium carboxymethyl cellulose), Polyanhydrides, Polymethyl merthacrylate, Polylactides, Polyglycolides, Carbomer, Gellan gum, Sodium alginate, Acrylic copolymers, Glyceryl monostearate, Zein, Cholesterol, Agarose, Chitosan, Xanthan gum, Polyethylene glycol (PEG), Gelatin, Povidone, Natural gum. The pellets may also contain Glidants (Flow Aids), Disintegrants, Lubricants, Adjuvants, Antibiotic Preservatives, etc. Suitable Glidants include: Precipitated silica, Fumed silica. Suitable Disintegrants include: Sodium starch glycolate, Crospovidone, Croscarmellose sodium. Suitable Lubricants include: Stearic acid, Magnesium stearate, Calcium stearate, Sodium stearyl fumarate, Glyceryl monostearate, Triglyceride esters. Suitable Adjuvants include but are not limited to: Aluminum hydroxide, Saponin, Dimethyl dioctadecyl ammonium bromide (DDA bromide), Bacterial extracts. If antibiotics are to be included, pellets may, for example, include the following: Penicillin, Streptomycin, Gentamicin, Polymyxin B, Amphotericin B, Nystatin, Tetracycline, Neomycin.

Preferably, the inert filler is lactose, and the functional filler hydroxypropyl cellulose (3%-30% of the pellet by weight). The glidant preferred is precipitated silica (0.5% of the pellet weight). The most satisfactory lubricant used to date would be calcium stearate at 2% of the pellet weight. Adjuvants include levels from 1%-20% of the pellet weight and may be aluminum hydroxide, DDA bromide or another suitable adjuvant. A suitable antibiotic preservative included at a <1% of pellet weight would be gentamicin.

All of the above may also be incorporated into the payload of ballistic implants as previously explained. We next describe the identification chip.

The choices of frequency of the identification chip are largely the choices of the manufacturer. In the U.S., common frequencies range from 100 kHz to 400 kHz, with the most preferred use being at 125 kHz, and in some instances 134.2 kHz, the latter being the most common frequency in Europe. It is desirable that the identification chips comply with the International Standards Organization (ISO) standards in order to have the largest compatible frequency opportunities with-available scanners. It is also preferable that in the injection system that the passive electronic device, i.e. the electronic identification chip, be the first implant, followed by the medicament doses. This is for both ease of packaging and ease of administration. Preferably the capsule of the electronic identification microchip is biologically inert and is one that passes Food And Drug Administration safety and efficacy standards.

As those skilled in the art know, once the microchip is implanted in the animal, the microchip remains inactive until read with a scanner. The scanner sends a low radio frequency signal to the chip providing the power needed by the microchip to send its unique code back to the scanner with the animals I.D. number. A single chip (M) in the drawings, normally lasts the life of the animal. The biological medicament doses which follow the chip in implantation are absorbed into the animal's system leaving only the chip. However, the chip identifies the animal; and, based upon data known before the dosing, the precise medicaments and the order of dosing of the medicaments that the animal has received. One example of an inert material useful for the capsule is soda lime glass which is known for compatibility with living tissue. The glass is hermetically sealed to keep its moisture out. It will normally last the life of an animal.

From the above description, it can be seen that the invention accomplishes all of its stated objectives.

## Claims

1. A dosing pellet magazine (22) for use in inoculating an animal, comprising; a plurality of connected pellet dosing columns (28), each of said columns being loaded with an electronic identification microchip and at least one biologically active dosing pellet (32), with said pellet being coded to represent a particular biologically active medicament, **characterised in that** the microchip has a convex end that is complementary to a concave surface of the at least one biologically active dosing pellet provided next to and behind the microchip in the column.

2. The dosing pellet magazine of claim 1 wherein the pellets (32) are loaded with the doses all in the same order.

3. The dosing pellet magazine of claim 1 wherein the animal (10) is selected from the group consisting of cattle, swine, horses, cats, dogs, sheep, goats, rabbits and birds.

4. The dosing pellet magazine of claim 1 wherein the pellet (32) is from 3% to 30% by weight of functional filler.

5. The dosing pellet magazine of claim 1 wherein the pellet (32) is color coded.

6. A method of packaging an electronic identification chip and biologically active implants, comprising: selecting a plurality of biologically active medicaments for implant dosing; coding each selected medicament with a unique code to represent the selected medicament; shaping the medicament to a pellet (32), placing the medicament pellet in a pellet magazine (22) with an electronic identification microchip having a convex surface, with the medicaments in a prearranged order; consistently using the same code scheme for packaging and instructional materials used with the packaged electronic identification microchip and pellet implant doses, **characterised in that** the micro chip has a convex end that is complementary to a concave surface of the medicament pellet provided next to and behind the microchip in the pellet magazine.

7. The method of claim 6 wherein the pellets (32) are loaded with the doses all in the same order.

8. The method of claim 6 wherein the pellet (3) is color coded.

## Patentansprüche

1. Dosierungspelletmagazin zur Verwendung bei der Impfung eines Tieres, umfassend: eine Vielzahl von miteinander verbundenen Pelletdosierschächten (28), wobei die Schächte jeweils mit einem elektronischen Identifikations-Mikrochip und wenigstens einem biologisch aktiven Dosierpellet (32) befüllt sind, wobei das genannte Pellet codiert ist, so dass es ein bestimmtes biologisch aktives Medikament repräsentiert, **dadurch gekennzeichnet, dass** der Mikrochip ein konvexes Ende hat, das mit einer konkaven Oberfläche des wenigstens einen biologisch aktiven Dosierpellets komplementär ist, das neben oder hinter dem Mikrochip in dem Schacht bereitgestellt ist.

2. Dosierpelletmagazin nach Anspruch 1, wobei die Pellets (32) jeweils mit allen Dosen in der gleichen Reihenfolge befüllt sind.

3. Dosierpelletmagazin nach Anspruch 1, bei dem das Tier (10) aus der Gruppe bestehend aus Rindern, Schweinen, Pferden, Katzen, Hunden, Schafen, Ziegen, Kaninchen und Vögeln ausgewählt ist.

4. Dosierpelletmagazin nach Anspruch 1, bei dem das Pellet (32) zu 3 bis 30 Gewichts-% aus funktionellem Füllstoff besteht.

5. Dosierpelletmagazin nach Anspruch 1, bei dem das Pellet (32) farbcodiert ist.

6. Verfahren zur Verpackung eines elektronischen Identifikations-Chips und biologisch aktiver Implantate, umfassend: Auswählen einer Vielzahl von biologisch aktiven Medikamenten zur Implantat-Dosierung, Codieren jedes gewählten Medikaments mit einem eindeutigen Code, so dass es das gewählte Medikament repräsentiert, Formen des Medikaments zu einem Pellet (32), Einlegen des Medikamentpellets mit den Medikamenten in einer bestimmten Reihenfolge in ein Pelletmagazin (22) mit einem elektronischen Identifikations-Chip, der eine konvexe Oberfläche hat, einheitliches Verwenden desselben Codessystems für Verpackungs- und Anweisungsmaterial, das mit dem/den verpackten elektronischen Identifiktions-Chip und Pelletimplantatdosen verwendet wird, **dadurch gekennzeichnet, dass** der Mikrochip ein konvexes Ende hat, das mit einer konkaven Oberfläche des Medikamentpellets komplementär ist, das in dem Pelletmagazin neben und hinter dem Mikrochip bereitgestellt ist.

7. Verfahren nach Anspruch 6, wobei die Pellets (32) jeweils mit allen Dosen in der gleichen Reihenfolge befüllt sind.

8. Verfahren nach Anspruch 6, bei dem das Pellet (32) farbcodiert ist.

## Revendications

1. Magasin de pellets de dosage (22) destiné à être utilisé lors de l'inoculation d'un animal, comprenant une pluralité de colonnes de dosage de pellets raccordées (28), chacune desdites colonnes étant chargée d'une micropuce d'identification électronique et d'au moins un pellet de dosage biologiquement actif (32), alors que ledit pellet est codé de façon à représenter un médicament spécifique biologiquement actif, **caractérisé en ce que** la micropuce présente une extrémité convexe laquelle est complémentaire d'une surface concave dudit au moins un pellet de dosage biologiquement actif prévu à côté de la micropuce située dans la colonne, ou se trouvant derrière celle-ci.

2. Magasin de pellets de dosage selon la revendication 1, les pellets (32) étant chargés de doses qui sont toutes dans le même ordre.

3. Magasin de pellets de dosage selon la revendication 1, l'animal (10) étant sélectionné parmi le groupe composé des espèces suivantes à savoir : bétail, porcs, chevaux, chats, chiens, moutons, chèvres, lapins et oiseaux.

4. Magasin de pellets de dosage selon la revendication 1, le pellet (32) étant constitué d'une charge fonctionnelle de 3 % à 30 % en poids.

5. Magasin de pellets de dosage selon la revendication 1, le pellet (32) ayant un codage couleur.

6. Procédé de conditionnement d'une puce d'identification électronique et d'implants biologiquement actifs comprenant les opérations consistant à : sélectionner une pluralité de médicaments biologiquement actifs en vue d'un dosage par implant ; coder chaque médicament sélectionné avec un code inédit pour représenter le médicament sélectionné ; façonner le médicament pour lui donner la forme d'un pellet (32) ; placer le pellet de médicament dans un magasin de pellets (22) avec une micropuce d'identification électronique présentant une surface convexe, alors que les médicaments se trouvent dans un ordre pré-agencé ; utiliser de façon cohérente le même plan de codage pour le conditionnement et les documents d'instruction utilisés en conjonction avec la micropuce d'identification électronique conditionnée et les doses d'implant des pellets, **caractérisé en ce que** la micropuce présente une extrémité convexe laquelle est complémentaire d'une surface concave du pellet de médicament prévu à côté de la micropuce située dans le magasin de pellets, ou se trouvant derrière celle-ci.

7. Procédé selon la revendication 6, les pellets (32) étant chargés de doses qui sont toutes dans le même ordre.

8. Procédé selon la revendication 6, le pellet (32) ayant un codage couleur.
